# EUROPÄISCHE PATENTANMELDUNG

(11) **EP 4 442 292 A1**
(43) Veröffentlichungstag der Anmeldung: **09.10.2024**
(21) Anmeldenummer: 24168060.2
(22) Anmeldetag: 02.04.2024
(51) Int. Cl.: A61M 1/16

(54) **VERFAHREN ZUM AUTOMATISCHEN KLASSIFIZIEREN EINES DIALYSATORS UND VERFAHREN ZUM BETREIBEN EINER DIALYSEMASCHINE MIT EINEM DIALYSATOR**

(30) Priorität: 05.04.2023 DE 102023108778
(71) Anmelder: B. Braun Avitum AG, 34212 Melsungen (DE)
(72) Erfinder: Janik, Waldemar, 34212 Melsungen (DE)
(74) Vertreter: Patentanwälte Ruff, Wilhelm, Beier, Dauster & Partner mbB

(57) **Zusammenfassung**

Verfahren zum, insbesondere prädialytischen, automatischen Klassifizieren eines Dialysators (1), mit den Schritten:
- Betreiben des Dialysators (1) bei unterschiedlichen Betriebsbedingungen,
- Erfassen von Betriebsvariablen bei den unterschiedlichen Betriebsbedingungen, und
- Zuordnen des Dialysators (1) zu einer Dialysator-Klasse in Abhängigkeit von den gemessenen Betriebsvariablen.

## Beschreibung

Der Erfindung liegt die Aufgabe zugrunde, ein Verfahren zum, insbesondere prädialytischen, automatischen Klassifizieren eines Dialysators zur Verfügung zu stellen, das ein möglichst einfaches und zuverlässiges automatischen Klassifizieren des Dialysators ermöglicht. Der Erfindung liegt die weitere Aufgabe zugrunde, ein Verfahren zum Betreiben einer Dialysemaschine mit einem Dialysator zur Verfügung zu stellen, das eine an die ermittelte Klasse des Dialysators angepasste Betriebsart ermöglicht.

Das Verfahren dient zum, insbesondere prädialytischen, automatischen Klassifizieren eines Dialysators bzw. zum Bestimmen des Typs des Dialysators.

Das Verfahren weist die Schritte auf: Betreiben des Dialysators bei unterschiedlichen Betriebsbedingungen, Erfassen von Betriebsvariablen bzw. Betriebsgrößen des Dialysators bei den unterschiedlichen Betriebsbedingungen, und Zuordnen des Dialysators zu einer vorgegebenen Dialysator-Klasse in Abhängigkeit von den gemessenen Betriebsvariablen.

In einer Ausführungsform unterscheiden sich die verschiedenen Dialysator-Klassen in mindestens einer der folgenden Eigenschaften: einem Dialysator-Volumen, Eigenschaften einer Dialysator-Membran, und einer herstellerspezifischen Modellzuordnung. Die herstellerspezifische Modellzuordnung ist beispielsweise die herstellerspezifische Bezeichnung bzw. der herstellerspezifische Typ des Dialysators.

In einer Ausführungsform weist das Betreiben des Dialysators bei den unterschiedlichen Betriebsbedingungen den Schritt auf: Verändern einer Eigenschaft eines Dialysat-Fluids auf der Dialysat-Seite des Dialysators, insbesondere an einem Dialysateingang des Dialysators. Das Erfassen der Betriebsvariablen bei den unterschiedlichen Betriebsbedingungen weist den Schritt auf: Messen einer Zeitdauer, bis sich die Eigenschaft des Dialysat-Fluids an einem Dialysatausgang des Dialysators in Reaktion auf das bewirkte Verändern der Eigenschaft des Dialysat-Fluids auf der Dialysat-Seite des Dialysators um ein vorgegebenes absolutes oder relatives Maß verändert hat, beispielsweise um 40 %, 50 % oder 60 % verändert hat. Der Schritt des Veränderns der Eigenschaft des Dialysat-Fluids auf der Dialysat-Seite des Dialysators kann ein kontinuierliches oder mehrstufiges Verändern der Eigenschaft des Dialysat-Fluids umfassen. Beispielsweise kann die Eigenschaft des Dialysat-Fluids von einem ersten Zustand auf einen zweiten Zustand und dann wieder zurück auf den ersten Zustand verändert werden, wobei der sich ergebende zeitliche Verlauf der Eigenschaft des Dialysat-Fluids zum Klassifizieren ausgewertet wird.

In einer Ausführungsform ist die Eigenschaft des Dialysat-Fluids die elektrische Leitfähigkeit des Dialysat-Fluids und/oder die Absorption, insbesondere die wellenlängenspezifische Absorption, des Dialysat-Fluids.

In einer Ausführungsform weist das Verändern der Eigenschaft des Dialysat-Fluids auf der Dialysat-Seite des Dialysators die Schritte auf: Fördern eines Test-Fluids mit einer ersten elektrischen Leitfähigkeit durch eine Blutseite des Dialysators, Fördern des Dialysat-Fluids mit einer zweiten elektrischen Leitfähigkeit, die sich von der ersten elektrischen Leitfähigkeit unterscheidet, durch die Dialysat-Seite des Dialysators, Unterbrechen des Förderns des Dialysat-Fluids durch die Dialysat-Seite des Dialysators für eine vorgegebene Zeitdauer, und Wiederaufnehmen des Förderns des Dialysat-Fluids durch die Dialysat-Seite des Dialysators. Das Erfassen der Betriebsvariablen bei den unterschiedlichen Betriebsbedingungen weist den Schritt auf: Messen eines zeitlichen Leitfähigkeitsverlaufes des Dialysat-Fluids an einem Dialysatausgang des Dialysators. Test-Fluid im Kontext der vorliegenden Anmeldung ist hierbei ein Fluid, das sich hinsichtlich mindestens einer Eigenschaft vom Dialysat-Fluid unterscheidet.

In einer Ausführungsform weist das Unterbrechen des Förderns des Dialysat-Fluids durch die Dialysat-Seite des Dialysators für eine vorgegebene Zeitdauer den Schritt auf: Umleiten des strömenden Dialysat-Fluids vorbei an der Dialysat-Seite des Dialysators für die vorgegebene Zeitdauer, wobei während der vorgegebenen Zeitdauer das Test-Fluid mit der ersten elektrischen Leitfähigkeit weiter durch die Blutseite des Dialysators gefördert wird.

In einer Ausführungsform wird aus dem gemessenen zeitlichen Leitfähigkeitsverlauf und/oder aus dem gemessenen zeitlichen Absorptionsverlauf des Dialysat-Fluids an dem Dialysatausgang des Dialysators ein Dialysator-Volumen abgeschätzt. Die Zuordnung des Dialysators zu einer Dialysator-Klasse kann in Abhängigkeit von dem abgeschätzte Dialysator-Volumen erfolgen.

In einer Ausführungsform weist das Betreiben des Dialysators bei den unterschiedlichen Betriebsbedingungen den Schritt auf: Verändern einer Ultrafiltrationsrate des Dialysators von einem ersten Wert auf einen zweiten Wert. Das Erfassen der Betriebsvariablen bei den unterschiedlichen Betriebsbedingungen weist den Schritt auf: Messen eines Transmembrandrucks bei dem zweiten Wert.

In einer Ausführungsform werden aus dem gemessenen Transmembrandruck Eigenschaften einer Dialysator-Membran abgeschätzt. Die Zuordnung des Dialysators zu einer Dialysator-Klasse kann in Abhängigkeit von den abgeschätzten Eigenschaften der Dialysator-Membran erfolgen.

In einer Ausführungsform wird basierend auf dem abgeschätzten Dialysator-Volumen und basierend auf den abgeschätzten Eigenschaften der Dialysator-Membran eine herstellerspezifische Modellzuordnung vorgenommen. Die Zuordnung des Dialysators zu einer Dialysator-Klasse kann in Abhängigkeit der herstellerspezifischen Modellzuordnung erfolgen.

Das erfindungsgemäße Verfahren zum Betreiben einer Dialysemaschine mit einem Dialysator weist die Schritte auf: Klassifizieren des Dialysators mittels eines oben beschriebenen Verfahrens und Einstellen von Betriebsparametern der Dialysemaschine in Abhängigkeit davon, welcher Dialysator-Klasse der Dialysator zugeordnet worden ist.

In einer Ausführungsform ist ein eingestellter Betriebsparameter ein Dialysatvolumenstrom durch den Dialysator, und/oder ein Blutvolumenstrom durch den Dialysator, und/oder eine Ultrafiltrationsrate, und/oder eine Substitutionsrate. Ein weiterer Betriebsparameter kann die Dauer der Dialysebehandlung sein.

Dialysatoren unterscheiden sich unter anderem in Form, Größe und den Membraneigenschaften. Vor der Therapie wird abhängig vom Patienten und dessen Gesundheitszustand ein entsprechender Dialysator ausgewählt.

Erfindungsgemäß wird der ausgewählte Dialysator automatisch klassifiziert, wodurch die Auswahl des Dialysators überprüft werden kann und dessen klassenabhängige Parameter für weitere Anwendungen gespeichert werden können.

Zur Durchführung des Verfahrens können beispielsweise Leitfähigkeits- und Druckmessungen in verschiedenen Zuständen der Dialysemaschine durchgeführt werden. Zur Dialysator-Identifizierung bzw. Klassifizierung können Methoden des Maschinellen Lernens verwendet werden, insbesondere in Form einer Support Vector Machine.

Zur messtechnischen Identifizierung des eingesetzten Dialysators kommen herkömmliche Dialysemaschinen in Frage, die beispielsweise über Leitfähigkeitssonden am Dialysateingang und Dialysatausgang, eine Ultrafiltrationseinrichtung (wie z.B. eine Pumpe am Dialysatausgang) sowie Drucksensoren auf Dialysat- und Blutseite verfügen.

Die Erfindung wird nachfolgend durch Bezugnahme auf die Zeichnungen detailliert beschrieben. Hierbei zeigt:
- Fig. 1: schematisch eine Dialysemaschine mit einem zu klassifizierenden Dialysator,
- Fig. 2: schematisch einen absoluten Leitfähigkeitsverlauf eines Dialysat-Fluids über der Zeit, einen normierten Leitfähigkeitsverlauf des Dialysat-Fluids über der Zeit, eine Ultrafiltrationsrate über der Zeit und einen Transmembrandruck über der Zeit bei unterschiedlichen Betriebsbedingungen des Dialysators,
- Fig. 3: eine Signalbreite eines Leitfähigkeits-Messsignals aufgetragen über einer Membranoberfläche unterschiedlicher Dialysatoren, und
- Fig. 4: einen Transmembrandruck aufgetragen über die Membranoberfläche unterschiedlicher Dialysatoren bei unterschiedlichen Ultrafiltrationsraten.

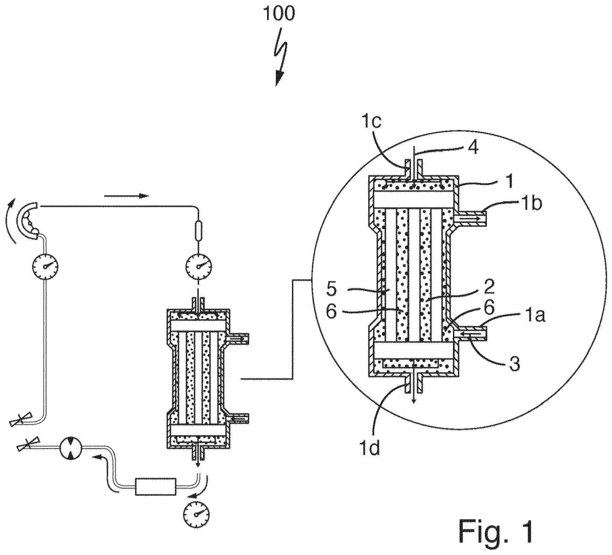

Fig. 1 zeigt exemplarisch und hoch schematisch eine Dialysemaschine 100 mit einem zu klassifizierenden Dialysator 1. Der Dialysator 1 weist herkömmlich auf einer Dialysatseite 6 einen Dialysateingang 1a und einen Dialysatausgang 1b auf, wobei zwischen Dialysateingang 1a und Dialysatausgang 1b ein Dialysat-Fluid 3 strömt.

Der Dialysator 1 weist weiter eine herkömmliche Dialysator-Membran 2 auf.

Der Dialysator 1 weist weiter herkömmlich auf einer Blutseite 6 einen Bluteingang 1c und einen Blutausgang 1d auf, wobei zwischen Bluteingang 1c und Blutausgang 1d im Betrieb des Dialysators 1 das zu reinigende Blut strömt.

Hinsichtlich des grundsätzlichen Aufbaus und der notwendigen Komponenten von Dialysatoren sei auch auf die einschlägige Fachliteratur verwiesen. Es versteht sich, dass Fig. 1 die Dialysemaschine lediglich schematisch darstellt und selbstverständlich weitere, nicht dargestellte Komponenten aufweist.

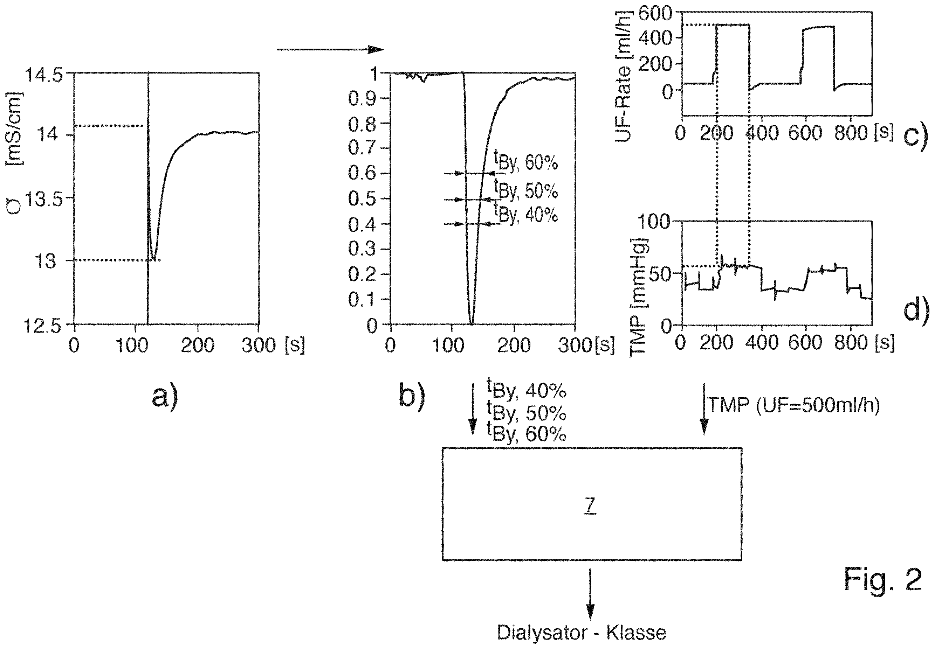

Das erfindungsgemäße Verfahren wird nachfolgend unter Bezugnahme auf die Figuren 2 bis 4 ausführlich beschrieben.

Fig. 2 a) zeigt hierbei schematisch einen absoluten Leitfähigkeitsverlauf des Dialysat-Fluids 3 am Dialysatausgang 1b über der Zeit, Fig. 2 b) einen normierten Leitfähigkeitsverlauf des Dialysat-Fluids 3 am Dialysatausgang 1b über der Zeit, Fig. 2 c) eine Ultrafiltrationsrate über der Zeit und Fig. 2 d) einen Transmembrandruck über der Zeit.

Das Verfahren weist in einer Ausführungsform beispielsweise folgende, insbesondere prädialytisch, ausgeführte Schritte auf:
1. Fördern einer ersten Flüssigkeit bzw. eines Test-Fluids 4 mit einer ersten Leitfähigkeit durch die Blutseite 5 des zu klassifizierenden Dialysators 1.
2. Fördern einer zweiten Flüssigkeit bzw. des Dialysat-Fluids 3 mit einer zweiten Leitfähigkeit, die sich von der ersten unterscheidet, durch die Dialysatseite 6 des zu klassifizierenden Dialysators 1.
3. Aktivieren einer Bypass-Phase, wobei die erste Flüssigkeit bzw. das Test-Fluid 4 weiterhin durch die Blutseite 6 des Dialysators 1 gefördert wird und die zweite Flüssigkeit bzw. das Dialysat-Fluid 3 am Dialysator 1 vorbeigeleitet wird.
4. Deaktivieren der Bypass-Phase, beispielswiese nach 60 Sekunden, und Messen eines Leitfähigkeitsausschlags, siehe Fig. 2 a) am Dialysatausgang 1b des Dialysators 1.
5. Normierung des Leitfähigkeitsverlaufs im Diagramm zwischen dem Leitfähigkeitsextremum und dem Leitfähigkeitswert vor Beendigung bzw. vor Beginn des Bypasses, siehe Fig. 2 b).
6. Ermittlung einer sich ergebenden Signalbreite des Leitfähigkeitsausschlags bei 40% *(tBy,40%),* 50% (*tBy*,50%) und/oder 60% (*tBy*,60%) des Signalausschlags.
7. Änderung der Ultrafiltrationsrate von einem ersten Wert (z.B. 50 ml/h) auf einen zweiten Wert (z.B. 500 ml/h), siehe Fig. 2 c).
8. Bestimmung des Transmembrandrucks (*TMP*) über der Zeit, insbesondere bei dem zweiten Wert, siehe Fig. 2 d).
9. Speisung eines Klassifikators 7 mit den ermittelten Werten *tBy*,40%*, tBy*,50%*, tBy*,60%*,* und *TMP.*
10. Ausgabe des mittels des Klassifikators 7 klassifizierten Dialysators 1.

Schritt 8 kann auch vor Schritt 3 erfolgen.

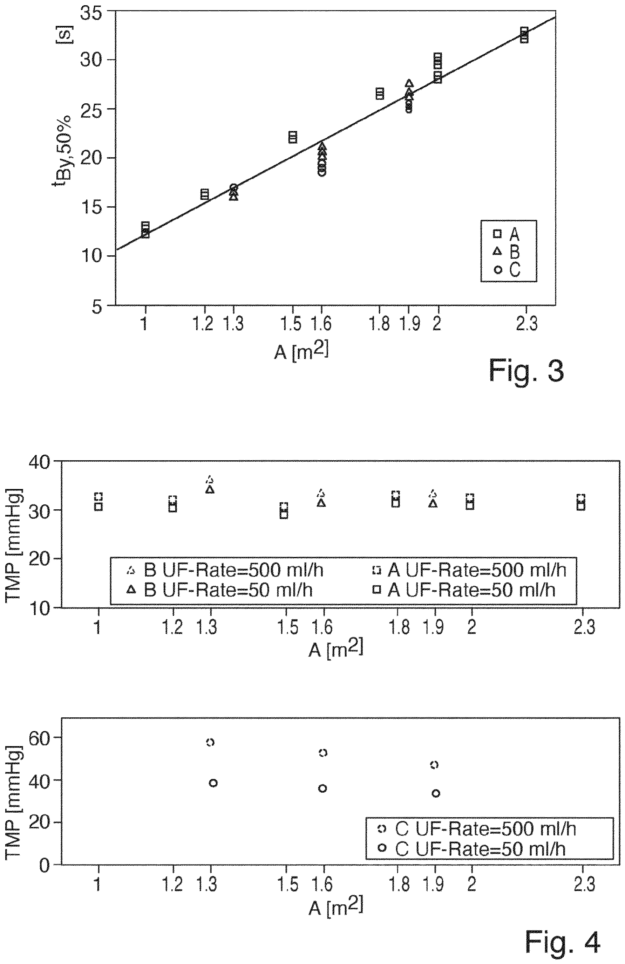

Fig. 3 zeigt die Signalbreite *tBy*,50% aufgetragen über die Membranoberfläche für unterschiedliche Dialysatoren A, B und C. Hierbei ist ein linearer Zusammenhang zu erkennen, siehe die abgebildete Regressionsgerade, so dass geschlussfolgert werden kann, dass eine größere Signalbreite auf einen größeren Dialysator hindeutet. Ein ähnliches Bild zeigt sich für die Signalbreiten bei 40% und 60% (nicht dargestellt).

Fig. 4 zeigt einen Transmembrandruck TMP aufgetragen über die Membranoberfläche A der unterschiedlichen Dialysatoren A, B und C bei unterschiedlichen Ultrafiltrationsraten von 500 ml/h bzw. 50 ml/h.

In der oberen Abbildung von Fig. 4 zeigt sich, dass der Transmembrandruck TMP der sogenannten High Flux Dialysatoren A und B nahezu unabhängig von der Ultrafiltrationsrate ist. In der unteren Abbildung von Fig. 4 ist dagegen zu erkennen, dass der Transmembrandruck TMP des Low Flux Dialysators C von der Ultrafiltrationsrate abhängt. So ist der Transmembrandruck TMP für niedrige Ultrafiltrationsraten niedriger als für hohe Ultrafiltrationsraten.

Durch Berechnung des Korrelationskoeffizienten nach Pearson wurde festgestellt, dass die Dialysatorgröße bzw. das Dialysator-Volumen am besten von den zuvor genannten drei Signalbreiten *tBy* repräsentiert wird. Analog hat sich ergeben, dass der Transmembrandruck TMP bei einer Ultrafiltrationsrate von 500 ml/h sehr gut die Eigenschaft High Flux oder Low Flux Dialysator repräsentiert.

Somit ergeben sich als Merkmale für den Klassifikator 7 die drei Signalbreiten *tBy*,40%*, tBy*,50% und *tBy*,60% sowie der Transmembrandruck TMP bei einer höheren Ultrafiltrationsrate UF.

Da die Signalbreiten neben dem Dialysator auch von dem Dialysatfluss abhängen, kann der Dialysatfluss entweder im Rahmen der Klassifizierung auf einen Standardwert von beispielsweise 500 ml/min eingestellt werden oder ebenfalls als Merkmal dem Klassifikator 7 zugeführt werden.

Aufgrund der allgemein guten Vorhersagewahrscheinlichkeit, der Flexibilität, der geringen Tendenz zum Auswendiglernen, der schnellen Klassifizierung von neuen Daten und des geringen Speicherplatzes kann als Klassifizierungsalgorithmus insbesondere die Support Vector Machine (SVM) verwendet werden. Selbstverständlich sind auch andere Klassifikatoren 7 denkbar.

Falls als erste Flüssigkeit bzw. Test-Fluid 4 eine Priminglösung beispielsweise in einem Beutel zirkuliert wird, kann das Verfahren bereits vor Dialysebeginn, d.h. prädialytisch, angewendet werden. Auch während einer laufenden Therapie ist das Verfahren problemlos durchführbar. Die erste Flüssigkeit bzw. das Test-Fluid 4 ist dann das Patientenblut.

Sobald der Dialysator 1 klassifiziert worden ist, können weitere Verfahren, die als Inputparameter die Klasse bzw. den Typ des Dialysators 1 verwenden, angewendet werden. Auch eine Optimierung der Behandlung ist denkbar, indem beispielsweise der Dialysatfluss auf die Klasse des Dialysators 1 abgestimmt wird.

Es ist außerdem denkbar, Dialysatoren bestimmter Hersteller zu erkennen, indem beispielsweise die Signalbreiten und der Transmembrandruck TMP mit auf einem Speichermedium hinterlegten Werten abgeglichen werden.

Zusätzlich oder alternativ zu einer Leitfähigkeitsmessung zur Klassifizierung, ist auch die Messung einer optischen Größe am Dialysatausgang 1b möglich, beispielsweise die Messung der Absorption, da auch hier die Form des Signals abhängig von der Dialysatorgröße ist. Das gleiche gilt für die Temperatur.

Zusätzlich oder alternativ zu den Signalbreiten können auch die Flächen der Signalverläufe betrachtet werden.

Die Bypassphase beträgt vorzugsweise 60 Sekunden. Sowohl kürzere als auch längere Bypässe sind jedoch ebenfalls denkbar.

Zusätzlich oder alternativ zu einer Bypassschaltungen können auch Leitfähigkeitsänderungen am Dialysateingang 1a vorgenommen werden, da die Dauer bis zur anschließenden Leitfähigkeitsänderung am Dialysatausgang 1b volumenabhängig und somit dialysatorabhängig ist.

Darüber hinaus sind auch Kombinationen dieser Merkmale möglich.

Ist die Unterscheidung zwischen Low Flux und High Flux Dialysator irrelevant, so kann der Schritt der Bestimmung des Transmembrandrucks entfallen. Dann reichen die Signalbreiten zur Ermittlung der Dialysatorgröße bzw. der Membranoberfläche aus.

## Patentansprüche

1. Verfahren zum, insbesondere prädialytischen, automatischen Klassifizieren eines Dialysators (1), mit den Schritten:
- Betreiben des Dialysators (1) bei unterschiedlichen Betriebsbedingungen,
- Erfassen von Betriebsvariablen bei den unterschiedlichen Betriebsbedingungen, und
- Zuordnen des Dialysators (1) zu einer Dialysator-Klasse in Abhängigkeit von den gemessenen Betriebsvariablen.

2. Verfahren nach Anspruch 1, **dadurch gekennzeichnet, dass**
- sich die verschiedenen Dialysator-Klassen in mindestens einer der folgenden Eigenschaften unterscheiden:
- einem Dialysator-Volumen,
- Eigenschaften einer Dialysator-Membran (2), und
- einer herstellerspezifischen Modellzuordnung.

3. Verfahren nach einem der vorhergehenden Ansprüche, **dadurch gekennzeichnet, dass**
- das Betreiben des Dialysators (1) bei den unterschiedlichen Betriebsbedingungen die Schritte aufweist:
- Verändern einer Eigenschaft eines Dialysat-Fluids (3) auf der Dialysat-Seite (6) des Dialysators (1), und
- das Erfassen der Betriebsvariablen bei den unterschiedlichen Betriebsbedingungen die Schritte aufweist:
- Messen einer Zeitdauer (tBy), bis sich die Eigenschaft des Dialysat-Fluids (3) an einem Dialysatausgang (1b) des Dialysators (1) um ein vorgegebenes Maß verändert hat.

4. Verfahren nach Anspruch 3, **dadurch gekennzeichnet, dass**
- die Eigenschaft des Dialysat-Fluids (3) die elektrische Leitfähigkeit des Dialysat-Fluids (3) ist, und/oder
- die Eigenschaft des Dialysat-Fluids (3) die Absorption, insbesondere die wellenlängenspezifische Absorption, des Dialysat-Fluids (3) ist.

5. Verfahren nach Anspruch 3 oder 4, **dadurch gekennzeichnet, dass**
- das Verändern der Eigenschaft des Dialysat-Fluids (3) auf der Dialysat-Seite (6) des Dialysators (1) die Schritte aufweist:
- Fördern eines Test-Fluids (4) mit einer ersten elektrischen Leitfähigkeit durch eine Blutseite (5) des Dialysators (1),
- Fördern des Dialysat-Fluids (3) mit einer zweiten elektrischen Leitfähigkeit, die sich von der ersten elektrischen Leitfähigkeit unterscheidet, durch die Dialysat-Seite (6) des Dialysators (1),
- Unterbrechen des Förderns des Dialysat-Fluids (3) durch die Dialysat-Seite (6) des Dialysators (1) für eine vorgegebene Zeitdauer, und
- Wiederaufnehmen des Förderns des Dialysat-Fluids (3) durch die Dialysat-Seite (6) des Dialysators (1), und
- das Erfassen der Betriebsvariablen bei den unterschiedlichen Betriebsbedingungen die Schritte aufweist:
- Messen eines zeitlichen Leitfähigkeitsverlaufes des Dialysats-Fluids (3) an einem Dialysatausgang (1b) des Dialysators (1).

6. Verfahren nach Anspruch 5, **dadurch gekennzeichnet, dass**
- das Unterbrechen des Förderns des Dialysat-Fluids (3) durch die Dialysat-Seite (6) des Dialysators (1) für eine vorgegebene Zeitdauer die Schritte aufweist:
- Umleiten des strömenden Dialysat-Fluids (3) vorbei an der Dialysat-Seite (6) des Dialysators (1) für die vorgegebene Zeitdauer, wobei während der vorgegebenen Zeitdauer das Test-Fluid (4) mit der ersten elektrischen Leitfähigkeit weiter durch die Blutseite (5) des Dialysators (1) gefördert wird.

7. Verfahren nach Anspruch 5 oder 6, **dadurch gekennzeichnet, dass**
- aus dem gemessenen zeitlichen Leitfähigkeitsverlauf und/oder aus dem gemessenen zeitlichen Absorptionsverlauf des Dialysat-Fluids (3) an dem Dialysatausgang (1b) des Dialysators (1) ein Dialysator-Volumen abgeschätzt wird.

8. Verfahren nach einem der vorhergehenden Ansprüche, **dadurch gekennzeichnet, dass**
- das Betreiben des Dialysators (1) bei den unterschiedlichen Betriebsbedingungen die Schritte aufweist:
- Verändern einer Ultrafiltrationsrate des Dialysators (1) von einem ersten Wert auf einen zweiten Wert, und
- das Erfassen der Betriebsvariablen bei den unterschiedlichen Betriebsbedingungen die Schritte aufweist:
- Messen eines Transmembrandrucks bei dem zweiten Wert.

9. Verfahren nach Anspruch 8, **dadurch gekennzeichnet, dass**
- aus dem gemessenen Transmembrandruck Eigenschaften einer Dialysator-Membran (2) abgeschätzt werden.

10. Verfahren nach Anspruch 7 und 9, **dadurch gekennzeichnet, dass**
- basierend auf dem abgeschätzten Dialysator-Volumen und basierend auf den abgeschätzten Eigenschaften der Dialysator-Membran (2) eine herstellerspezifische Modellzuordnung vorgenommen wird.

11. Verfahren zum Betreiben einer Dialysemaschine (100) mit einem Dialysator (1), mit den Schritten:
- Klassifizieren des Dialysators (1) mittels eines Verfahrens nach einem der vorhergehenden Ansprüche und
- Einstellen von Betriebsparametern der Dialysemaschine (100) in Abhängigkeit davon, welcher Dialysator-Klasse der Dialysator (1) zugeordnet worden ist.

12. Verfahren nach Anspruch 11, **dadurch gekennzeichnet, dass**
- ein eingestellter Betriebsparameter ein Dialysatvolumenstrom durch den Dialysator (1) ist, und/oder ein Blutvolumenstrom durch den Dialysator (1) ist, und/oder eine Ultrafiltrationsrate ist, und/oder eine Substitutionsrate ist.
